# EUROPÄISCHE PATENTSCHRIFT

(11) **EP 1 121 146 B1**
(45) Veröffentlichungstag und Bekanntmachung des Hinweises auf die Patenterteilung: **30.06.2004**
(21) Anmeldenummer: 99953778.0
(22) Anmeldetag: 12.10.1999
(51) Int. Cl.: A61K 38/48, A61K 31/7048, A61P 37/00

(54) **BEEINFLUSSUNG VON HYPERAKTIVEN T-ZELLEN DURCH PROTEOLYTISCHE ENZYME**
UTILIZATION OF PROTEOLYTIC ENZYMES TO INFLUENCE HYPERACTIVE T CELLS
UTILISATION D'ENZYMES PROTEOLYTIQUES POUR INFLUER SUR DES LYMPHOCYTES T HYPERACTIFS

(30) Priorität: 13.10.1998 DE 19847114
(43) Veröffentlichungstag der Anmeldung: 08.08.2001
(73) Patentinhaber: MUCOS Pharma GmbH & Co., D-82538 Geretsried (DE)
(72) Erfinder: RANSBERGER, Karl, D-82402 Seeshaupt (DE); STAUDER, Gerhard, D-82538 Geretsried (DE)
(74) Vertreter: Grünecker, Kinkeldey, Stockmair & Schwanhäusser Anwaltssozietät
(86) Internationale Anmeldenummer: PCT/EP1999/007634
(87) Internationale Veröffentlichungsnummer: WO 2000/021547

(56) Entgegenhaltungen:
- WO-A-96/00082
- DE-A- 4 130 221
- DE-A- 4 302 060
- DE-A- 19 726 255
- TARGONI OLEG ET AL: "Modulation of the activation threshold for autoreactive T cells via systemic enzyme therapy with Phlogenzym." 11TH EUROPEAN CONGRESS ON MULTIPLE SCLEROSIS JOURNAL OF NEUROIMMUNOLOGY 1995, Nr. SUPPL. 1, 1995, Seite 66 XP000906964 ISSN: 0165-5728
- WALD M ET AL: "Proteolytic enzymes prevent B16 melanoma metastasizing in C57B16 mice." ECCO 10: THE EUROPEAN CANCER CONFERENCE;VIENNA, AUSTRIA; SEPTEMBER 12-16, 1999 EUROPEAN JOURNAL OF CANCER SEPT., 1999, Bd. 35, Nr. SUPPL. 4, 1999, Seite S371 XP000906897 ISSN: 0959-8049

## Beschreibung

Die vorliegende Erfindung betrifft ein Verfahren zur Beeinflussung von hyperaktiven T-Zellen durch proteolytische Enzyme sowie die Verwendung proteolytischer Enzyme zur Beeinflussung von hyperaktiven T-Zellen gemäß der Ansprüche.

Bei bestimmten Infektionen oder Krebserkrankungen oder auch bei renalen Krankheiten kommt es zu einer Verschiebung von in Homöostase befindlichen verschiedenen Subpopulationen von Lymphozyten, Granulozyten und Monozyten. Normalerweise ist der Organismus in der Lage, im Prozeß einer Genesung die Verteilungsgleichgewichte der Subpopulationen wieder herzustellen.

Bei chronischen Erkrankungen, insbesondere bei Autoimmunerkrankungen und der damit verbundenen permanenten Belastung des Immunsystems (z.B. durch Immunkomplexe und im Zusammenhang mit chronisch persistierenden Virusinfektionen wie AIDS), kommt es im Verlauf der Erkrankung zu einer vom Organismus nicht mehr kompensierbaren Verschiebung sowohl der Gleichgewichte der Zellpopulationen als auch der Expressionsdichte einiger spezifischer Antigene. Allgemein spricht man hier von einer unkontrollierten Aktivierung des Immunsystems. Für die Untersuchung solcher Autoimmunerkrankungen stehen Tiermodelle zur Verfügung, so dient z.B. die Induktion von allergischer Encephalomyelits in Mäusen als Modell für Multiple Sklerose, wobei die Mäuse nach Verabreichung von Gehimsubstanz bzw. bestimmter Peptide eine Autoimmunreaktion zeigen, welche sich als Paralyse einzelner Körperteile messen läßt.

In der letzten Zeit würden eine Reihe von Studien angefertigt, die anzudeuten scheinen daß die Entwicklung der oben genannten Krankheiten in starkem Ausmaß von der Aktivierung und Expansion spezifischer T-Lymphozyten abhängt Sogar bei denjenigen renalen Krankheiten, die im wesentlichen durch Antikörper vermittelt werden, konnte eine eindeutige Beteiligung der T-Zellen festgestellt werden.

In Versuchen, bei denen Tiere mit Antigenen immunisiert wurden, konnte festgestellt werden, daß spezifische T-Zellen aktiviert wurden und Cytokine freisetzten.

Cytokine sind wichtige Stoffwechselstoffe bei der Auslösung verschiedener Kranheitszustände. Es handelt sich um kleine Proteine mit Malekulargewichten von 8000 bis 30000, wobei jedes Cytokin eine eigene Aminosäuresequenz und Zelloberflächenrezeptoren aufweist. Sie werden von einer Vielzahl von verschiedenen Zelltypen hergestellt und wirken auf fast jedes Gewebe und Organsystem. Die Namen, die den verschiedenen Cytokinen gegeben wurden, folgen keinem logischen System, sondern entsprechen vielmehr ihrer biologischen Eigenschaft. IL-1 (Interleukin -1) und TNF (Tumornekrosefaktor) sind die primären proinflammatorischen Cytokine, und außerdem wirken diese zwei Cytokine in einer synergistischen Weise bei der Induktion von Entzündungen, Schock und Tod.

Die oben beschriebene Immunreaktion der T-Zellen mit Freisetzung von Cytokinen kann sich z.B. als multiple Sklerose, Typ I Diabetes, rheumatoide Arthritis oder Glomerulonephritis manifestieren, oder auch als Transplantatabstoßung.

Nach dem bisherigen Kenntnisstand erhärtet sich die Annahme, daß die Modulation von T-Zelloberflächenmolekülen des Immunsystems ein besonderes Feld der Medikation darstellen kann und daß sich hyperaktive T-Zellen auf diesem Wege beeinflussen lassen.

Der vorliegenden Erfindung lag daher das technische Problem zugrunde, ein Verfahren zur Beeinflußung von hyperaktiven T-Zellen anzugeben.

Diese Aufgabe wird durch die in Anspruch 1 angegebene Erfindung gelöst Vorteilhafte Weiterbildungen sind in den Unteransprüchen angegeben.

Gemäß Anspruch 1 ist die Verwendung von mindestens einem proteolytischen Enzym und Rutosid zur Herstellung eines Medikamentes zur Beeinflussung von hyperaktiven T-Zellen, wobei die hyperaktiven Zellen ausgewählt sind aus Transplantat-spezifischen, Allergen-spezifischen und Virus-spezifischen T-Zellen, vorgesehen. Das Auftreten hyperaktiver T-Zellen ist für viele immunvermittelte Entzündungserkrankungen, wie z.B. die oben genannten Krankheiten, charakteristisch. Durch die Verwendung proteolytischer Enzyme ist daher eine Verbesserung der entsprechenden Krankheitssymptome bis hin zum vollständigen Abklingen derselben möglich.

Die Beeinflussung von hyperaktiven T-Zellen durch Proteasen funktioniert Vermutlich auf folgendem Wege:

Eine der Proteasen, Trypsin als Beispiel, wird im Körper einer enteropankreatischen Rezirkulation unterzogen. Nachdem es vom exokrinen Pankreas sekretiert wurde, wird es vom Verdauungstraktlumen resorbiert und ins Blut transportiert, von wo es wieder in den Pankreas resekretiert wird. Trypsin ist ein normaler Bestandteil des Serums. Ungefähr 10 % von oral verabreichtem Trypsin enden schließlich im Blut Wie andere Serumproteasen, ist Serumtrypsin an eine Antiprotease gebunden, das α2-Makroglobulin. In diesem Komplex ist Trypsin von einer Autoverdauung und auch von einem Abbau durch andere Serumproteasen geschützt Es behält jedoch seine enzymatische Aktivität. An Entzündungsstellen, an denen die vaskuläre Permeabilität lokal erhöht ist, tritt Serumprotein in den interstitiellen Bereich ein. Auf diese Weise wird Trypsin an der Stelle der Entzündung angereichert.

Es wird angenommen, däß Trypsin insbesondere die Zelloberflächenmoleküle CD4, CD44 und B7-1 modifiziert, die wichtige Regulatoren der T-Zellimmunreaktion sind. Anders als bei seiner Aktion im Verdauungstrakt, wo Trypsin ein breites Spektrum von Substanzen abbauen kann, scheint seine Wirkung nur auf einige Oberflächenmoleküle, unter anderem die gerade genannten drei, begrenzt zu sein. Dies kann z.B. daran liegen, daß in einem entzündeten Gewebe die Bindungsstellen für das Trypsin durch Glykosylierung maskiert sind oder im Proteinkem versteckt liegen.

Die oben genannten drei Zelloberflächenmoleküle CD4, CD44 und B7-1 sind alle an der Regulierung des Grenzwerts für eine T-Zellaktivierung beteiligt T-Zellen greifen nur das Antigen an, das ihnen auf Antigen-präsentierenden Zellen dargeboten wird. Bei diesem Erkennungsprozeß bindet der Antigenrezeptor der T-Zelle (TCR) spezifisch an einen Haupthistokompatibilitätskomplex (MHC-Komplex) und ein bestimmtes Peptidfragment des nativen Antigens. Diese Zusammenwirkung bestimmt die Spezifität der T-Zellerkennung. Es gibt einen Grenzwert, bei dessen Überschreitung T-Zellen aktiviert werden. Die Definition für die Bedingungen, unter denen T-Zellaktivierung auftritt , d.h. der minimalen Signalstärke, die von einer T-Zelle benötigt wird, um den Grenzwert für ihre Aktivierung zu überschreiten, ist wie folgt:

T-Zellaktivierung tritt auf, wenn eine minimale Anzahl an Bindungen (Ligationen) des T-Zellrezeptors zu einem MHC-Komplex und Antigen in einer definierten Zeitspanne überschritten wird. Diese Anzahl wird für T-Zellen als konstant angenommen und kann z.B. in der Größenordnung von 100 TCR Ligationen pro Zelle pro Sekunden liegen. Diese Zahl (X) definiert den T-Zellaktivierungsgrenzwert. Gemäß der aktuellen Definition benötigen T-Zellen mit hoher Affinität (T-Zellen, deren TCRs eine hohe Affinität für einen gegebenen MHC-Peptidkomplex besitzen) weniger Liganden (MHC: Nominalpeptidkomplexe) für die Aktivierung als T-Zellen, welche eine geringe Affinität für das Antigen haben.

Im Gegensatz dazu wird in der vorliegenden Erfindung davon ausgegangen, daß der T-Zellaktivierungsgrenzwert kein starres intrinsisches Merkmal der T-Zellen ist, sondern zusätzlich eine Funktion des Aktivierungsstadiums der T-Zelle und der damit verbundenen Expression von co-stimulierenden Molekülen ist. Demgemäß ist die Anzahl an T-Zellrezeptorligationen, die für Aktivierung benötigt wird, eine Funktion der vorangegangenen Interaktionen zwischen T-Zellen und Antigen, z.B. Zeitspanne seit der letzten Interaktion, Stärke und Häufigkeit der Interaktion mit Antigen und dem Zusammenspiel spezieller Cytokine oder anderer Mediatoren, die die Zellen zusätzlich stimulieren. Native T-Zellen, die noch keine Interaktion mit einem Antigen eingegangen sind, exprimieren wenige akzessorische Moleküle und benötigen mehr Antigen, um stimuliert zu werden, als normale ruhende T-Gedächniszellen (Pihlgren et al., J. Exp. Med., 184: 2141, 1996). Dies bedeutet, daß wenn für die Aktivierung von nativen Zellen die Zahl X als TCR-Ligationen pro Sekunde angenommen wird, diese Zahl für normale Gedächniszellen < X sein sollte. Diese Gedächniszellen sind durch frühere Interaktionen mit Antigenen prä-aktiviert worden, wobei das Antigen mit der Zeit aus dem Organismus ausgeschieden wurde und die Zellen in ein mehr ruhendes Stadium zurückkehrten. Sie behalten jedoch eine höhere Expression von akzessorischen Molekülen auf ihrer Oberfläche und benötigen geringere Antigenkonzentrationen für ihre Aktivierung.

Gemäß dem dieser Erfindung zugrundeliegenden Modell sind hyperaktive T-Zellen solche Zellen, die relativ viele akzessorische Moleküle auf ihrer Zelloberfläche tragen und kontinuierlich durch Wechselwirkung ihrer spezifischen T-Zellrezeptoren mit Antigenen stimuliert sind, wie z.B. Transplantat-spezifische, Allergen-spezifische oder Virus-spezifische T-Zellen, die jeweils bei Transplantationen, Allergien oder chronischen viralen Infektionen zu beobachten sind. Dabei gilt für hyperaktive T-Zellen, daß sie schon auf weniger als 1/3 der Menge an Antigen reagieren, die notwendig ist, um eine Reaktion in einer "normalen", d.h. nicht hyperaktiven T-Zelle auszulösen. Diese T-Zellreaktion ist durch Produktion von Cytokinen und Chemokinen sowie durch Proliferation oder zellvermittelte Zytolyse gekennzeichnet.

Es wird für diese Erfindung vorausgesetzt, daß hyperaktive T-Zellen sogar weniger TCR-Ligationen als normale Gedächniszellen für erneute Aktivierung benötigen, d.h. ihr Aktivierungsgrenzwert ist <<X, da hyperaktive T-Zellen mehr akzessorische Moleküle auf ihrer Oberfläche exprimieren, als dies bei normalen Gedächniszellen der Fall ist. Die Expression an akzessorischen Molekülen ist dabei proportional zum Präaktivierungsstadium der T-Zellen (Anzahl an akzessorischen Molekülen: ruhende/native T-Zellen < Gedächnis/aktivierte T-Zellen < Gedächnis/hyperaktivierte T-Zellen). Weiterhin ist die Anzahl an für T-Zellaktivierung benötigter TCR-Ligationen umgekehrt proportional zur Anzahl an exprimierten akzessorischen Molekülen (X: ruhende T-Zellen < Gedächniszellen < hyperaktivierte T-Zellen). Da die erhöhte Reaktionsbereitschaft von hyperaktiven T-Zellen aus ihrer erhöhten Expression an akzessorischen Molekülen resultiert, kann die Spaltung bzw. Neutralisation dieser überschüssigen akzsessorischen Moleküle mit Hilfe von Enzymen, z.B. Trypsin, die hyperaktiven T-Zellen funktionell den normalen Gedächnis-T-Zellen angleichen. Im Fall einer extensiven Neutralisation der akzessorischen Moleküle können hyperaktive T-Zellen funktionell sogar an native T-Zellen angeglichen werden. Nachdem der Aktivierungsgrenzwert für hyperaktive T-Zellen durch Enzymbehandlung angehoben wird, sollte die Dosis an vorhandenem Antigen (MHC/Peptidkomplexdichte auf Antigen präsentierenden Zellen), die ausreichend war, um T-Zellen in dem hyperaktiven Stadium zu erhalten, unter den Grenzwert für erneute T-Zellaktivierung sinken, wodurch den T-Zellen erlaubt wird, aus einem hyperaktivierten Stadium in ein ruhenderes Stadium zurückzukehren. Demgemäß sollten die T-Zellvermittelten Krankheitssymptome zurückgehen oder diese zumindest günstig beeinflussen.

Die anderen Proteasen wirken in ähnlicher Weise wie Trypsin.

In einer bevorzugten Ausführungsform wird als proteolytisches Enzym Trypsin, Bromelain, Papain oder eine Kombination derselben verwendet.

Die erfindungsgemäß verwendeten Enzyme lassen sich kostengünstig aus den folgenden Rohmaterialien isolieren.

Bromelain ist ein proteolytisch wirksames Enzym aus dem Preßsaft der Ananas und kann auch noch aus reifen Früchten isoliert werden.

Papain ist ein proteolytisches Enzym, das aus dem Milchsaft der unreifen, fleischigen Früchte des Melonenbaums Carica papaya gewonnen wird. Reines Papain ist ein kristallines Polypeptid mit einem MG. von 23350, das aus einer Kette von 212 Aminosäureresten mit 4 Disulfid-Brücken besteht; Sequenz und Raumstruktur sind bekannt. Papain wird vielfältig eingesetzt Aufgrund seiner Protein-spaltenden Eigenschaft als "Fleischzartmacher" oder "Mürbesalz", zum Klären von Bier, zur Brot- und Hartkeksherstellung, in der Lederzubereitung, in der Textil-Industrie zum Entbasten von Seide und zur Verhinderung von Wollverfilzung, in der Tabak-Industrie zur Qualitätsverbesserung, zur Rückgewinnung von Silber aus verbrauchtem photographischem Material, ferner in der Bakteriologie zur Pepton-Gewinnung. In der Medizin dient Papain bereits zur Unterstützung der enzymatischen Verdauung, zur enzymatischen Wundreinigung und als Zusatz zu Zahnprothese-Reinigungsmitteln. Für Spezialzwecke werden Papain-Präparate auch an Kunststoffpolymere oder Agarose trägergebunden angeboten. Papain ist auch als Katalysator zur Synthese von Oligopeptiden verwendet worden.

Trypsin ist ein proteolytisches Enzym, das ebenfalls im Pankreas gebildet wird und in Verbindung mit anderen Enzymen bereits therapeutisch eingesetzt wird. Es gehört zu den Serin-Proteinasen. Kristallines Trypsin hat ein MG von ca. 23300, ist in Wasser, nicht aber in Alkohol löslich, besitzt ein Wirkungsoptimum bei pH 7-9 und spaltet Peptid-Ketten spezifisch Carboxy-seitig der basischen Aminosäurereste L-Lysin und L-Arginin. Die räumliche Struktur des aus 223 Aminosäuren bestehenden Trypsins ist bekannt.

Eine besonders gute Wirksamkeit zeigt sich bei der Verwendung einer Kombination der Enzyme Bromelain, Papain und/oder Trypsin. Neben der bemerkenswerten und unerwarteten Wirkung dieser Enzyme hat die kombinierte Verwendung der genannten Enzyme weiterhin den Vorteil, daß auch bei einer Langzeitanwendung keine schädigende Nebenwirkungen auftreten.

Weiterhin wird zusätzlich Rutosid dem Medikament beigemischt. Rutosid ist ein Glycosid, das zu den Flavonoiden gehört.

Eine besonders gute Wirksamkeit hat die kombinierte Verwendung von 20 bis 100 mg Bromelain, 40 bis 120 mg Papain und 10 bis 50 mg Trypsin pro Dosiseinheit.

Ganz besonders bevorzugt ist eine Kombination von 90 mg Bromelain, 120 mg Papain und 100 mg Rutosid x 3H₂O.

In einer anderen bevorzugten Ausführungsform wird eine Kombination von 48 mg Trypsin, 90 mg Bromelain und 100 mg Rutosid x 3H₂O verwendet. Diese Kombination wird z.B. unter dem Namen Phlogenzym® von der Firma Mucos Pharma GmbH & Co. in Deutschland vertrieben.

In einer weiteren bevorzugten Ausführungsform werden 10 bis 100 mg, besonders bevorzugt 100 mg Rutosid x 3 H₂O pro Dosiseinheit verwendet

Das Medikament kann weiterhin alle üblichen Hilfs- und/oder Trägerstoffe enthalten.

Als Hilfs- und Trägerstoffe kommen z.B. Lactose, Magnesiumstearat, Stearinsäure, Talkum, Methacrylsäure, Copolymerisat Typ A, Shellack, Makrogol 6000, Dibutylphthalat, Vanillin, Titandioxid, weißer Ton, Polyindon, gelbes Wachs und Camaubawachs in Frage.

Die Erfindung betrifft weiterhin ein in vitro Verfahren zur Beeinflussung von hyperaktiven T-Zellen wobei die T-Zellen mit einem oder mehreren proteolytischen Enzymen und zusätzlich Rutosid in Kontakt gebracht werden.

Die folgenden Abbildungen, Tabellen und Beispiele sollen die Erfindung im einzelnen erläutern.

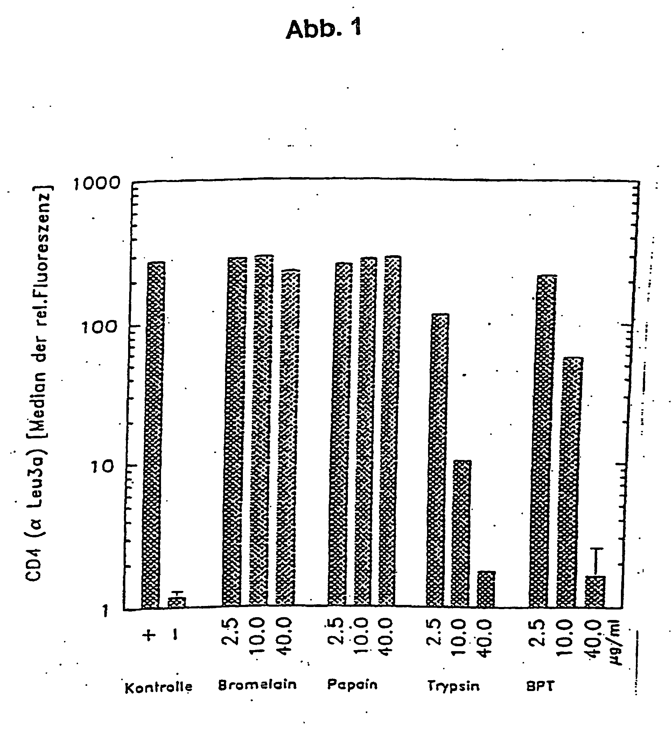

### Abb. 1 CD4 (Epitop Leu3a)-Modulation durch Proteasen

Angegeben ist der Median der relativen Fluoreszenzintensität der Targetzellen (Lymphozyten). Die Positivkontrolle (Referenz) sind unbehandelte Zellen, die mit dem für CD4 spezifischen, monoklonalen Antikörper inkubiert wurden. Die Negativkontrolle sind unbehandelte Zellen, die mit dem Antikörper-Isotyp inkubiert wurden. Die Inkubationszeit mit den Enzymen betrug 60 min. Dargestellt sind die Daten van Spender 2 (vgl. Tab. 1) als Mittelwert von Doppelbestimmungen und Standardabweichung.

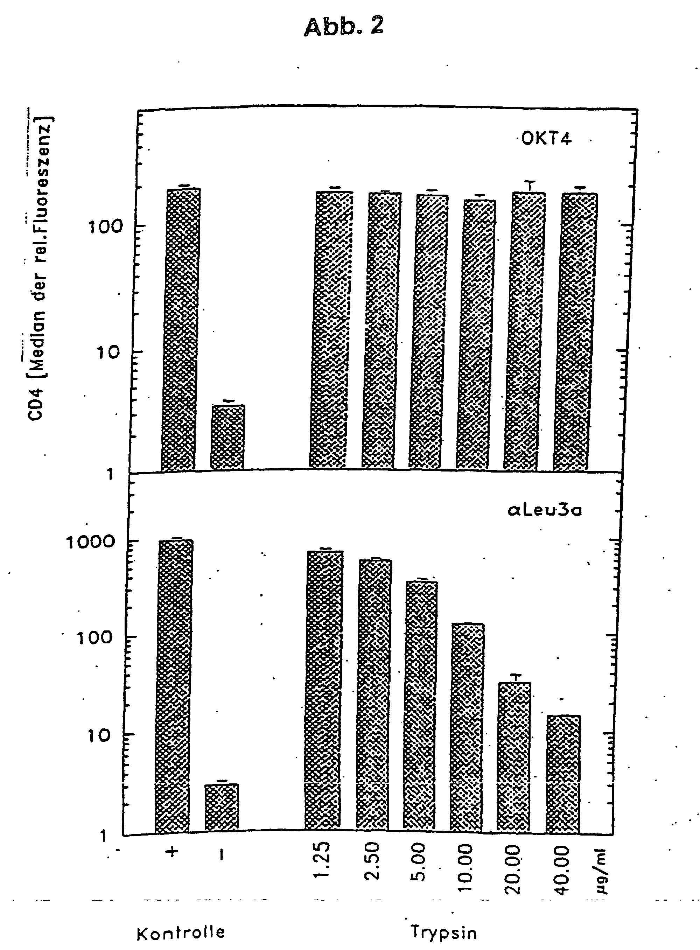

### Abb. 2 CD4 (Epitope OKT4 und Leu3a)-Modulation durch Trypsin

Angegeben ist der Median der relativen Fluoreszenzintensität der Targetzellen (Lymphozyten). Die Positivkontrolle (Referenz) sind unbehandelte Zellen, die mit dem für CD4 spezifischen monoklonalen Antikörper inkubiert wurden. Die Negativkontrolle sind unbehandelte Zellen, die mit dem Antikörper-Isotyp inkubiert wurden. Die Inkubationszeit mit den Enzymen betrug 60 min. Dargestellt sind die Daten von einem Spender (vgl. Tab. 2) als Mittelwert von Doppelbestimmungen und Standardabweichung.

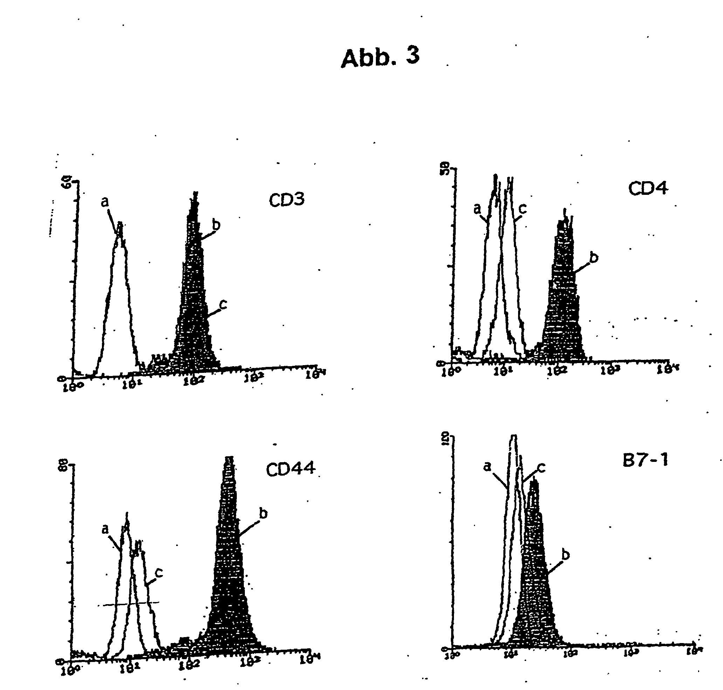

### Abb. 3 Expression von Oberflächenmolekülen nach Phlogenzym®behandlung in vitro

Der T-Zellhybridklon F. 10.9 (obere Figuren) und der B-Zell Tumorklon B220 (untere Figuren) wurden jeweils mit 50 µg/ml an Phlogenzym bei 37°C inkubiert und anschließend markiert.
a: Markierung der Isotypenkontrolle
b: Markierung von HEL-behandelten Kontrollzellen
c: Markierung nach Phlogenzym®behandlung

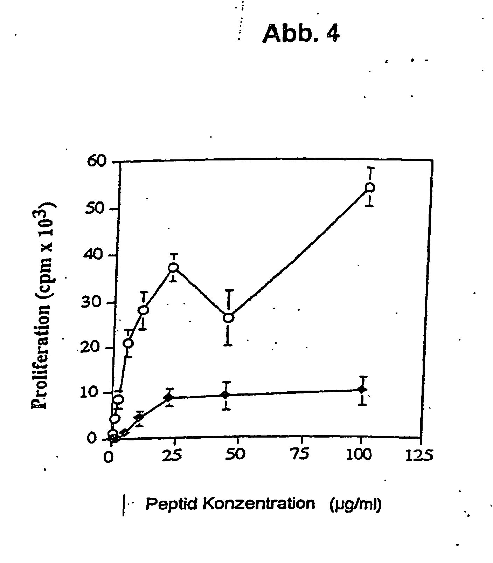

### Abb. 4 Proteolipidprotein (PLP):139-151 Peptid-spezifische proliferative "recall response" in Mäusen, die mit Phlogenzym® oder HEL gefüttert wurden

Die Mäuse wurden mit PLP:139-151 Peptid immunisiert und mit 41 mg an Phlogenzym® (geschlossene Symbole) oder Hühnereiweißlysozym (HEL; offene Symbole) gefüttert. Am Tag 30 wurden die Milzzellen in der Anwesenheit der angegebenen PLP-Peptidkonzentrationen auf ihre proliferative "recall response" getestet. Die Daten sind als Durchschnittswerte mit Standardabweichung von drei parallelen Kulturen einer Maus jeder Gruppe dargestellt und sind repräsentativ für fünf in jeder Gruppe getesteten Mäuse. Diese Ergebnisse wurden in zwei Experimenten reproduziert.

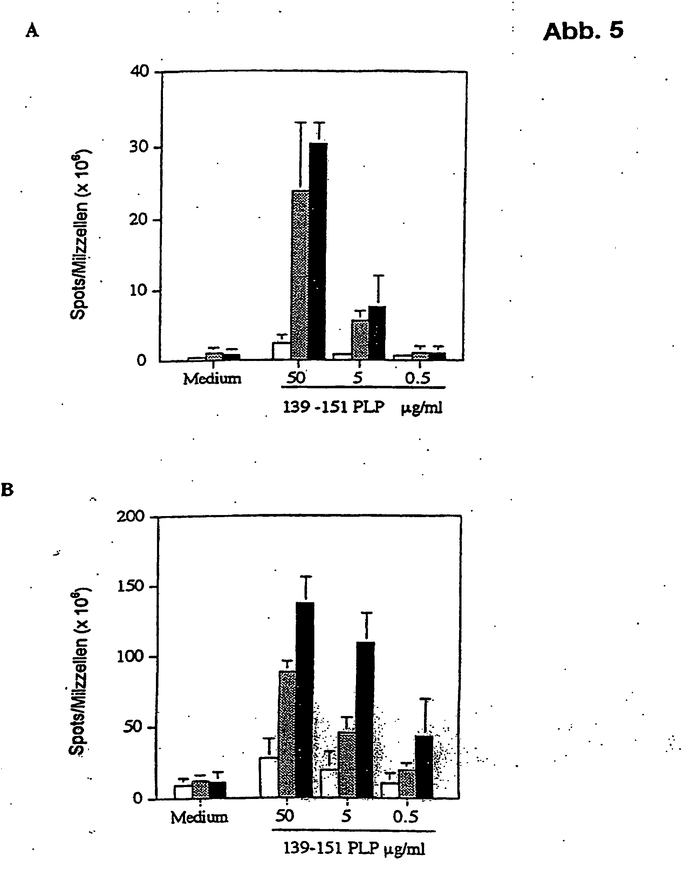

### Abb. 5 Cytokin "recall response" in Mäusen, die mit Phlogenzym und HEL als Kontrolle behandelt wurden

5 SJL-Mäuse pro Gruppe wurden mit PLP:139-151 Peptid immunisiert. Vom Tag der Immunisierung an wurden sie zweimal täglich durch künstliche Ernährung mit 42 mg an Phlogenzym oder einer äquivalenten Dosis an HEL gefüttert. Lymphzellen wurden in ELISA spot-Tests auf IL-5 (A) und IL-4 (B) Produktion in der Anwesenheit von Medium alleine oder PLP:139-151 Peptid in den angegebenen Konzentrsationen getestet.

Weiße Balken: kranke Kontrollmäuse, gefüttert mit 42 mg an HEL, getestet am Tag 21 graue Balken: nicht erkrankte Mäuse, gefuttert mit 42 mg an Phlogenzym, getestet am Tag 21
schwarze Balken: mit HEL behandelte Mäuse, getestet am Tag 60 nach Rekonvaleszenz von allergischer Encephalomyelitis.

Die Daten stellen den Durchschnittswert und die Standardabweichung von drei parallelen Kulturen einer Maus jeder Gruppe dar und sind repräsentativ für fünf Mäuse, die in diesem Experiment in jeder Gruppe getestet wurden. Die Ergebnisse wurden in drei Experimenten reproduziert.

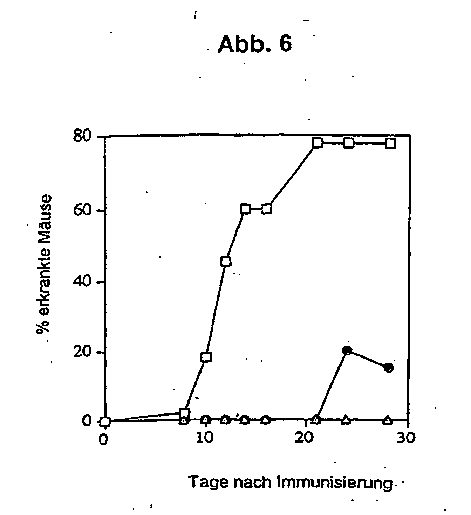

### Abb. 6 Ausbildung von allergischer Encephalomyelitis in Mäusen, die oral mit Phlogenzym® und HEL behandelt wurden

Aktive allergische Encephalomyelitis wurde in Gruppen von je 10 SJL-Mäusen pro Experiment durch PLP:139-151 Peptid-Immunisierung injiziert, während die Mäuse vom ersten Tag der Immunisierung an täglich zweimal durch künstliche Ernährung mit 8,2 mg (schwarze Kreise, 20 Mäuse) oder 41 mg (helle Kreise, 30 Mäuse) an Phlogenzym® gefüttert wurden. Kontrollmäuse (ebenfalls 10 Mäuse pro Experiment) wurden mit äquivalenten Mengen an HEL gefüttert (helle Quadrate). Da kein Unterschied zwischen den mit 8,2 mg Phlogenzym® und den 41 mg HEL gefütterten Gruppen bestand, wurden die Daten für alle insgesamt 50 Kontrollmäuse zusammen berechnet Die durchschnittliche Kranheitsrate lag für die Kontrollmäuse bei 3,7 und für die mit 8,2 mg Phlogenzym® behandelten Mäuse bei 1,6.

### Beispiel 1

### Material und Methoden

### 1. Material

### Zellkultur:

Es wurde das Zellkulturmedium RPMI 1640 mit folgenden Zusätzen verwendet: NaHCO₃ (Biochrom, 2 g/1); L-Glutamin (Biochrom, 2 mM), Na-Pyruvat (Biochrom, 1 mM), NaN₃ (Sigma, 0,01 %).

Im Falle einer Stimulation der Zellen wurde entweder Phythämagglutinin M (Biochrom, 5 µg/ml) oder γ-Interferon (100 U/ml) eingesetzt. Die Stimulation der Zellen erfolgte dabei über 1-3 Tage mit Zusatz von 10 % fötalem Kälberserum (Biochrom).

Als Targets für die zu untersuchenden Enzyme wurden frisch isolierte, humane, periphere mononukleäre Blutzellen (Citratblut) verwendet. Nach der üblichen Isolation mittels Ficoll wurden die Zellen mehrfach gewaschen und frisch bei den Experimenten eingesetzt.

### In vivo-Experimente:

Für in vitro-Experimente mit Phlogenzym® wurde der T-Zellklon F.10.9 und der B-Zellklon B220 verwendet. Bei in vivo-Experimenten mit Phlogenzym® (Mucos Pharma GmbH & Co. GmbH) bzw. Hühnereiweißlysozym (HEL; Sigma, St. Louis, MO) als Kontrolle wurden weibliche SJL-Mäuse (The Jackson Laboratories) im Alter von 6-10 Wochen eingesetzt.

Das zur Induktion von allergischer Encephalomyelitis in Mäusen verwendete Peptid Proteolipidprotein (PLP):139-151 (H-KISQAVHAAHAE-OH; Princecton Biomolecules, Columbus, OH) wurde mit vollständigem Complete Freud's Adjuvans (CFA; 2,5 mg/ml M. tuberculosis H37RA; Difco Laboratories, Detroit, MI) in unvollständigem Freud's Adjuvans (IFA; Gibco BRL, Grand Island, NY) vermischt, um eine Emulsion von 1 mg/ml zu ergeben, von welcher jeder Maus 50 µl (50 µg/Maus) subkutan injiziert wurden. Ab Tag 8 nach der Immunsierung wurden die Mäuse auf die Entwicklung paralytischer Symptome untersucht und der Schweregrad der Krankheit wurde gemäß der Standardskala gemessen.
Grad 1: relaxierter Schwanz
Grad 2: Hinterbeinschwäche
Grad 3: vollkommene Paralyse der Hinterbeine
Grad 4: Quadriplegie
Grad 5: Tod

Um die Ernährung paralysierter Mäuse Sicherzustellen, wurden in den Käfigen lange Wasserröhrchen eingesetzt und feste Nahrung in das Nest gelegt.

### 2. Proliferationstest

Dieser Test wurde wie in Lehmann et al., Nature, 1992, 358, 155-157 durchgeführt. Aus einem Milzorgan wurden Einzelzellsuspensionen hergestellt und 1 x 10⁶ dieser Zellen wurden jeweils in eine Vertiefung einer 96-Loch Flachbodenmicrotiterplatte in serumfreiem HL-1 Medium (Bio Whittaker, Walkersville, MD), ergänzt mit 1 mM L-Glutamin, ausgesät Antigene oder Peptide wurden zu einer Endkonzentration von 100 µg/ml zugesetzt. Während der letzten 18 h der 4-Tages-Kultur wurde ³H-Tymidin (1 µCi/Vertiefung) zugesetzt. Die Inkorporation der radioaktiven Markierung wurde durch Scintillationszählung gemessen.

### 3. Cytokin-Messung durch ELISA-Spot Tests

Die Methode wurde bereits von Forsthuber et al., Science (1996), 271, 1728-1730 beschrieben.

ImmunoSpot-Platten (Autoimmun Diagnostika, Beltsville, MD) wurden über Nacht mit den spezifisch IFN-γ oder IL-5-bindenden Antikörpern R46-A2(4 µg/ml) oder TRFKS- (5 µg/ml) in PBS beschichtet. Die Platten wurden mit 1 % BSA in PBS unspezifisch für 1 h bei Raumtemperatur geblockt und 4-mal mit PBS gewaschen. 1 x 10⁶ Milzzellen wurden alleine in HL-1 Medium oder mit Peptiden in spezifischen Konzentrationen ausptattiert und wurden im Falle von IFN-γ für 24 h und im Falle von IL-5 für 48 h kultiviert. Anschließend wurden die Zellen durch Waschen entfernt und der Antikörper für die Detektion (1 µg/ml XMG1.2-HRP für IFN-γ oder 4 µg/ml TRFK4 für IL-5) zugesetzt und über Nacht inkubiert.

Für IL-5 wurde das Antigen anti-IgG2a-HRP (Zymed) zugesetzt und für 2 h inkubiert. Der letzte an die Platte gebundene Antikörper wurde durch Zugabe von AEC visualisiert Für die Bewertung der Ergebnisse wurde ein Series 1 ImmunoSpot Image Analyzer (Autoimmun Diagnostika) eingesetzt.

### 4. Verwendete monoklonale Antikörper

Für die spezifische Erkennung der Oberflächenstrukturen der Leukozyten wurden monoklonale Antikörper verwendet. Diese erkennen auf den entsprechenden Antigenen jeweils ein definiertes Epitop, welches bei den untersuchten Antigenen nur ein einziges Mal in der Struktur vorkommt. In Übersicht 1 sind die untersuchten Oberflächenmarker, die monoklonalen Antikörper sowie die analysierten Targetzellen dargestellt.

Übersicht 1: Analysierte Oberflächenmarker, verwendete monoklonale Antikörper und Targetzellen der Enzymbehandlung

| Marker | Epitop AK-Bez. | Fluorochrom | Produzent | Target-Zelle |
|---|---|---|---|---|
| | | | | |
| CD4 | Leu3a | PE | B.D. | T-Lymphozyten |
| | OKT4 | FITC¹ | Ortho² | T-Lymphozyten |

| | | | | |
|---|---|---|---|---|
| ¹ Fluoreszeinisothiocyanat, grüne Fluoreszenz; | | | | |
| ² Ortho Diagnostics | | | | |

### 5. Inkubationsbedingungen

Die frisch isolierten und aufbereiteten Zellen wurden mit den Enzymen Bromelain, Papain und Trypsin (Arzneimittel-Inhaltsstoffe der Fa. Mucos Pharma GmbH & Co.) oder Phlogenzym® (vertrieben von der Fa. Mucos Pharma GmbH & Co.) und HEL (Hühnereiweißlysozym) mit den jeweils in den Legenden der Tabelle und Abbildung angegebenen Konzentration inkubiert. Bei der Mischung der drei Enzyme entsprach das Mischungsverhältnis 22,7 : 15,5 : 11,9 (Bromelain : Papain : Trypsin, bezogen auf 40 µg/ml, "BPT"). Es wurden drei Enzymkonzentrationen (40, 10, 2,5 µg/ml) für BPT untersucht und eine Konzentration von 50 µg/ml an Phlogenzym® eingesetzt. Die Inkubation fand in serumfreiem Medium bei 37°C statt.

Die Proteasen wurden unmittelbar vor der Inkubation angesetzt. In den Zellkulturmedien war 0,01 % Natriumazid enthalten. Durch diesen Zusatz werden die Zellen daran gehindert, während des Inkubationsprozesses oder der Waschvorgänge Rezeptormoleküle erneut zu exprimieren. Nach dem Auswaschen des Zellkulturmediums sind die Zellen wieder aktivierbar (nicht demonstriert).

Nach entsprechender Inkubation der Zellen mit den jeweiligen Enzymen, Auswaschen der Enzyme und der Markierung mit monoklonalen Antikörpern (nach Angaben der Hersteller) wurde sofort die Analyse der Oberflächenmarker vorgenommen.

### 6. Analytische Durchflußcytometrie

Alle Untersuchungen zur Modulation von Zelloberflächenmolekülen wurden mittels analytischer DurchflußCytometrie (FACSCAN, Fa. Becton Dickinson, Heidelberg) unter Verwendung einer gerätespezifischen Software (Lysis I) durchgeführt. Für Experimente mit Phlogenzym® wurde die Cell Quest Software (Becton Dickinson, Mountain View, CA) eingesetzt. Bei entsprechender Einstellung des Gerätes sowie der Mitführung von Referenzen, d.h. Zellen, die ohne Enzyme aber in derselben Prozedur behandelt wurden, erfolgte die Messung.

Für die monoklonalen Antikörper wurde eine entsprechende fluoreszenzkonjugierte Isotypenkontrolle mitgeführt. Hierbei handelt es sich um Maus-Immunglobulin, mit welchem die Kapazität der unspezifischen Bindung der Targetzellen durchflußcytometrisch erfaßt wird.

Pro Histogramm wurden 10 000 Zellen gemessen. Die jeweilige Zellpopulation wurde mit einem sogenannten "elektronischen Gate" separiert, in dem sich dann mindestens 3 500 Zellen befanden.

### 7. Darstellung der Ergebnisse

### 7.1 Proteolytische Spaltung von Oberflächenmolekülen durch Trypsin, Bromelain, Papain und BPT (Vergleichend)

Die Auswertung und Analyse der Daten erfolgte unabhängig vom Meßvorgang auf dem Durchflußcytometer mittels einer gerätespezifischen Software. Dabei wurden jeweils die Histogramme der Kontrollen, d. h. der unbehandelten Zellproben, mit den Histogrammen der enzymbehandelten Zellen verglichen.

Die Rohdarstellung umfaßt ein optisch eindrucksvolles, aber relativ unübersichtliches Histogramm, bei dem verschiedene Einzelmessungen übereinander gelagert abgebildet sind. Für diese Untersuchungen ist nicht der prozentuale Anteil einer Subpopulation an der gesamten Leukozytenpopulation die Meßgröße, sondern die relative Rezeptordichte, die sich als Fluoreszenzintensität darstellt.

Aus diesen Histogrammen lassen sich Daten ableiten, welche die relative Fluoreszenzintensität der Einzelmessung reflektieren. Diese ist ein Maß für die relative Rezeptor- oder Oberflächenmoleküldichte bei einer gemessenen Zellpopulation.

Die Säulengraphiken zeigen in logarithmischer Darstellung den Median der relativen Fluoreszenz (Abb. 1, 2). Es läßt sich so gegenüber der Referenz die Reduktion der Dichte des jeweiligen Oberflächenmoleküls in Abhängigkeit von der Enzymkonzentration darstellen.

In den Tabellen sind die Daten unabhängiger Experimente enthalten (Tabelle 1,2). Wird in der Tabelle beispielsweise ein Wert von 40 % angegeben, so bedeutet das, daß bei diesem Antigen 40 % aller Oberflächenmoleküle durch das Enzym so verändert ist, daß der spezifische monoklonale Antikörper sein Epitop nicht mehr erkennt Wird keine Reduktion beobachtet, so erscheint in den Tabellen der Wert "0". Die angegebene Prozentzahl drückt somit die Enzymleistung gegenüber den einzelnen Antigenen aus Werte bis zu 20 % werden im Einzelfall als nicht relevant angesehen.

Für eine Bewertung der Wirkung der Enzyme auf die einzelnen Antigene ist es günstig, einen geeigneten Vergleichsmaßstab zu wählen. Bis auf wenige Ausnahmen wurden alle Untersuchungen unter standardisierten Inkubationsbedingungen durchgeführt. Damit kann für diese experimentellen Bedingungen die aus früheren Forschungsberichten bekannte Halbeffektkonzentration angegeben werden.

Zur Berechnung der Halbeffektkonzentration wurden die Daten mittels nicht-linearer Regression ausgewertet Der Median der Fluoreszenzintensität versus eingesetzter Enzymkonzentration und Referenz werden zueinander in Beziehung gesetzt, und daraus läßt sich die Menge an Enzym berechnen, die zu einer 50%igen Reduktion der relativen Fluoreszenzintensität bzw. der in der Struktur veränderten Rezeptordichte führt.

In Tabelle 1 ist die CD4(Epitop Leu3a)-Modulation auf Lymphozyten durch die Proteasen; Bromelain, Papain, Trypsin und die Proteasemischung BPT dargestellt. Angegeben ist die prozentuale Reduktion des Medians der relativen Fluoreszenzintensität, die ein Maß der Enzymleistung ist. Die Inkubationszeit mit den Enzymen betrug 60 Min. Es sind die Ergebnisse von zwei Experimenten mit Zellen von zwei verschiedenen Spendern dargestellt.

| Enzym | Nr. | 40 µg/ml | 10 µg/ml | 2,5 µg/ml |
|---|---|---|---|---|
| Bromelain | 1 | 24,6 | 0 | 0 |
| | 2 | 16,2 | 0 | 0 |
| Papain | 1 | 2,5 | 3,2 | 0 |
| | 2 | 0 | 0 | 5,7 |
| Trypsin | 1 | 99,3 | 93,0 | 64,1 |
| | 2 | 99,4 | 96,2 | 57,5 |
| BPT | 1 | 98,3 | 49,3 | 23,0 |
| | 2 | 99,4 | 79,2 | 20,2 |

Tabelle 2 zeigt die Modulation der CD4-Epitope Leu3a und OKT4 durch Trypsin auf Lymphozyten. Angegeben ist die prozentuale Reduktion des Medians der relativen Fluoreszenzintensität, die ein Maß der Enzymleistung ist Die Inkubationszeit mit den Enzymen betrug 60 Min. Es sind die Daten von einem Spender dargestellt.

| Enzym | Epitop | 40 µg/ml | 20 µg/ml | 10 µg/ml |
|---|---|---|---|---|
| Trypsin | Leu3a | 98,5 | 96,7 | 87,1 |
| | OKT4 | 6,5 | 6,3 | 19,5 |
| | | | | |

| | Epitop | 5 µg/ml | 2,5 µg/ml | 1,25 µg/ml |
|---|---|---|---|---|
| Trypsin | Leu3a | 65,2 | 41,9 | 28,8 |
| | OKT4 | 13,3 | 10,8 | 9,3 |

### 7.2 Proteolytische Spaltung von Oberflächemolekülen durch Phlogenzym®

Es wurde untersucht, welche Zelloberflächemoleküle, die an der Interaktion zwischen T-Zellen und Antigen präsentierenden Zellen (APZ) beteiligt sind, von Phlogenzym® gespalten werden können. In Abb. 3 wird gezeigt, daß eine zweistündige Enzymbehandlung (50 µg/ml) von T- und B-Zellhybridomen die Erkennung von CD4, CD44 und B7-1 durch entsprechende FACS-Marker deutlich reduziert, während die CD3-Markierung nicht betroffen ist Ebenso war die Erkennung von MHC Klasse I, MHC Klasse II und LFA-1-Motekülen nach Inkubation mit Phtogenzym nicht verändert, während die Erkennung von ICAM-1 und B7-1-Zelloberflächenmolekülen leicht reduziert war. Diese Ergebnisse demonstrieren, daß Proteasen trotz ihrer ubiquitären Spaltungsmotive sehr selektiv Veränderungen der Zelloberflächenmoleküle verursachen können.

### 7.3 Rechtsverschiebung der Dosis-Wirkungskurve für T-Zellaktivierung in Mäusen, denen Phlogenzym oral zugeführt wurde

Da der Schwellenwert für die T-Zellaktivierung abhängig ist von der Anzahl an akzessorischen Molekülen sollte die Spaltung von CD4-, CD44- und B7-1- Molekülen eine Rechtsverschiebung der Dosiswirkungskurve für die T-Zellantwort verursachen. Es wurde daher die proliferative "recall response" auf Peptid PLP:193-151 in frisch isolierten Milzzellen an Mäusen getestet, welche mit diesem Peptid immunisiert wurden und welchen Phlogenzym® oder HEL über eine Sonde zur künstlichen Ernährung zugeführt wurde (41 mg Enzym/Maus, zweimal täglich). Die Ergebnisse sind in Abb. 4 dargestellt Die Antwort der Phlogenzym®-behandelten Mäuse betrug ca 10-40 % der Antwort, die bei Kontrollmäusen gemessen wurde. Zusätzlich war bei submaximalen Peptidkonzentrationen die Dosiswirkungskurve in der Phlogenzym®-behandelten Gruppe nach rechts verschoben. Dies zeigt, daß Phlogenzym® behandlung in vivo die T-Zellfunktion in einer Weise modifiziert, die einem erhöhten Schwellenwert für T-Zellaktivierung, welche aus der Spaltung akzessorischer Moleküle, die an der T-Zell/APZ-Interaktion beteiligt sind, entspricht

### 7.4 T_{helfer} 2-Zell-Switching der autoantigenen spezifischen T-Zellantwort in Phlogenzym®-behandelten Mäusen

Schwache T-Zellaktivierung mit niedriger Avidität induziert gewöhnlich eine T_{Helfer}2-Zell-Antwort (Th2). Da B7-1-Moleküle die Th1-Entwicklung fördern, könnte die Spaltung dieser Moleküle einen Th2-Switch der Autoimmun-T-Zellantwort bedingen. Um diese Moglichkeit zu testen, wurden Mäuse mit dem Peptid PLP:139-151 immunisiert und die Cytokinqualität der "recall response" auf dieses Peptid untersucht. Die Mäuse wurden entweder mit zweimal 41 mg/Maus/Tag an Phlogenzym® oder HEL gefüttert und 21 Tage nach der Immunsierung getestet; zu einem Zeitpunkt, an dem die meisten der HEL-gefütterten Kontrollmäuse, jedoch keine der Phlogenzym®-behandelten Mäuse allergische Encephalomyelitis entwickelt hatten. Milzzellen der erkrankten HEL gefütterten Mäuse zeigten keine signifikante IL-4 (Abb. 5A) oder IL-5 (Abb. 5B) Produktion in Antwort auf das PLP:139-151 Peptid im Vergleich zu der Medium-Kantrolle (weiße Balken), zeigten jedoch eine starke IFN-γ-"recall response" auf dieses Peptid. Die Phlogenzym®-behandelten Mäuse, welche nicht erkrankt waren, zeigten dieselbe starke PLP:139-151 spezifische IL-4 und IL-5 Produktion (graue Balken). Die IFN-γ-"recall response" war nicht signifikant verschieden zwischen den beiden Gruppen. Daher wurde in den Phlogenzym®-behandelten Mäusen die Th2-Antwort verstärkt. Diese Cytokin-"recall response" vom Th2-Typ wurde ebenfalls drei Monate nach Immunisierung in den Kontrollmäusen gesehen, zu einem Zeitpunkt, zu dem sich die Mäuse von allergischer Encephalomyelitis erholt hatten (schwarze Balken).

### 7.5 Orale Verabreichung von hydrolytischen Enzymen verhindert Entwicklung von allergischer Encephalomyelitis

Jeder Maus wurden über eine Sonde zur künstlichen Ernährung 8,2 mg Phlogenzym® zweimal täglich gefüttert, was nach Gewichtskorrektur der äquivalenten menschlichen Dosis entspricht. Kontrollmäusen wurde dieselbe Menge an HEL verabreicht Zusammen mit der Enzymverabreichung fand die Immunisierung mit dem krankheitsinduzierenden PLP-Peptid 139-151 statt und wurde bis zum Ende des Experimentes fortgesetzt. Die Mäuse wurden täglich daraufhin untersucht, ob sie paralytische Symptome, welche für allergische Encephalomyelitis charakteristisch sind, entwickeln. 39 der 50 Kontrollmäuse (78%; Abb. 6; rechteckige Symbole) entwickelten Krankheitssymptome mit einer durchschnittlichen Krankheitsrate von 3,7, während lediglich 4 der 20 mit Phlogenzym® behandelten Mäuse (20%; Abb. 6; kreisförmige Symbole) Symptome mit einer durchschnittlichen Krankheitsrate von 1,6 entwickelten. Da Mäuse einen schnelleren Metabolismus als Menschen haben, wurde die Phlogenzym® dosis erhöht, um zu testen, ob dies einen stärkeren Effekt hervorrufen kann. Es wurde festgestellt, daß die 5-fache Dosis (41 mg/Maus, zweimal täglich) die Entwicklung von allergischer Encephalomyelitis in insgesamt 30 getesteten Mäusen vollständig inhibierte (Abb. 6; dreieckige Symbole). Bei kontinuierlicher Behandlung hatten die Mäuse keine Symptome in der einmonatigen Beobachtungsperiode, woraus geschlossen wurde, daß orale Verabreichung von Phlogenzym® einen starken krankheitsinhibierenden Effekt hat

## Patentansprüche

1. Verwendung von mindestens einem proteolytischen Enzym und Rutosid zur Herstellung eines Medikamentes zur Beeinflussung von hyperaktiven T-Zellen, wobei die hyperaktiven Zellen ausgewählt sind aus Transplantat-spezifischen, Allergen-spezifischen und Virus-spezifischen T-Zellen.

2. Verwendung gemäss Anspruch 1, **dadurch gekennzeichnet, dass** als proteolytisches Enzym Trypsin, Bromelain oder Papain oder eine Kombination von einem oder mehreren dieser Enzyme verwendet wird.

3. Verwendung nach einem oder mehreren der Ansprüche 1 bis 2, **dadurch gekennzeichnet, dass** 20 bis 100 mg Bromelain, 40 bis 120 Papain und 10 bis 50 mg Trypsin pro Dosiseinheit verwendet werden.

4. Verwendung nach einem oder mehreren der Ansprüche 1 bis 3, **dadurch gekennzeichnet, dass** 90 mg Bromelain, 120 mg Papain und 100 mg Rutosid pro Dosiseinheit verwendet werden.

5. Verwendung nach einem oder mehreren der Ansprüche 1 bis 2, **dadurch gekennzeichnet, dass** 90 mg Bromelain, 48 mg Trypsin und 100 mg Rutosid pro Dosiseinheit verwendet werden.

6. *In vitro* Verfahren zur Beeinflussung von hyperaktiven Zellen ausgewählt aus Transplantat-spezifischen, Allergen-spezifischen und Virus-spezifischen T-Zellen, wobei die hyperaktiven T-Zellen mit einem oder mehreren proteolytischen Enzymen und zusätzlich Rutosid in Kontakt gebracht werden.

## Claims

1. Use of at least one proteolytic enzyme and of rutoside for preparing a drug for influencing hyperactive T cells, wherein the hyperactive cells are selected from transplant-specific, allergen-specific and virus-specific T cells.

2. Use according to claim 1, **characterized in that** trypsin, bromelain or papain or a combination of several of said enzymes is used as the proteolytic enzyme.

3. Use according to one or several of claims 1 to 2, **characterized in that** 20 to 100 mg bromelain, 40 to 120 mg papain and 10 to 50 mg trypsin are used per dose unit.

4. Use according to one or several of claims 1 to 3, **characterized in that** 90 mg bromelain, 120 mg papain and 100 mg rutoside are used per dose unit.

5. Use according to one or several of claims 1 to 2, **characterized in that** 90 mg bromelain, 48 mg trypsin and 100 mg rutoside are used per dose unit.

6. *In vitro* method for influencing hyperactive cells selected from transplant-specific, allergen-specific and virus-specific T cells, wherein the hyperactive T cells are contacted with one or several proteolytic enzymes and, additionally, with rutoside.

## Revendications

1. Utilisation d'au moins une enzyme protéolytique et de rutoside pour la préparation d'un médicament pour influencer les cellules T hyperactives, les cellules hyperactives étant choisies parmi les cellules T spécifiques d'un greffon, spécifiques d'un allergène et spécifiques d'un virus.

2. Utilisation selon la revendication 1, **caractérisée en ce qu'**on utilise comme enzyme protéolytique de la trypsine, de la bromélaïne ou de la papaïne ou une combinaison d'une ou de plusieurs de ces enzymes.

3. Utilisation selon l'une ou plusieurs des revendications 1 à 2, **caractérisée en ce qu'**on utilise 20 à 100 mg de bromélaïne, 40 à 120 de papaïne et 10 à 50 mg de trypsine par unité de dosage.

4. Utilisation selon l'une ou plusieurs des revendications 1 à 3, **caractérisée en ce qu'**on utilise 90 mg de bromélaïne, 120 mg de papaine et 100 mg de rutoside par unité de dosage.

5. Utilisation selon l'une ou plusieurs des revendications 1 à 2, **caractérisée en ce qu'**on utilise 90 mg de bromélaïne, 48 mg de trypsine et 100 mg de rutoside par unité de dosage.

6. Procédé in vitro pour influencer des cellules hyperactives choisies parmi les cellules T spécifiques d'un greffon, spécifiques d'un allergène et spécifiques d'un virus, les cellules T hyperactives étant mises en contact avec une ou plusieurs enzymes protéolytiques et en outre du rutoside.
